# FASCICULE DE BREVET EUROPEEN

(11) **EP 2 696 745 B1**
(45) Date de publication et mention de la délivrance du brevet: **15.11.2017**
(21) Numéro de dépôt: 12712671.2
(22) Date de dépôt: 10.04.2012
(51) Int. Cl.: A61B 3/12, A61B 5/00

(54) **DISPOSITIF D'AIDE À LA DÉTECTION DE CARACTÉRISTIQUES ANATOMIQUES D'AU MOINS UNE PORTION D'UN TISSU. PROCÉDÉ D'AIDE À LA DÉTECTION DE CARACTÉRISTIQUES ANATOMIQUES D'AU MOINS UNE PORTION D'UN TISSU**
VORRICHTUNG ZUR UNTERSTÜTZUNG BEIM NACHWEIS ANATOMISCHER MERKMALE VON MINDESTENS EINEM TEILS EINES GEWEBES UND VERFAHREN ZUR UNTERSTÜTZUNG BEIM NACHWEIS ANATOMISCHER MERKMALE VON MINDESTENS EINEM TEIL EINES GEWEBES
DEVICE FOR ASSISTING IN DETECTING ANATOMICAL FEATURES OF AT LEAST A PORTION OF A TISSUE AND METHOD FOR ASSISTING IN DETECTING ANATOMICAL FEATURES OF AT LEAST A PORTION OF A TISSUE

(30) Priorité: 15.04.2011 FR 1153306
(43) Date de publication de la demande: 19.02.2014
(73) Titulaire: Université Pierre et Marie Curie (Paris 6), 75005 Paris (FR)
(72) Inventeur: PAQUES, Michel, 75012 Paris (FR); MREJEN, Sarah, 75003 Paris (FR)
(74) Mandataire: Parzy, Benjamin Alain
(86) Numéro de dépôt international: PCT/EP2012/056428
(87) Numéro de publication internationale: WO 2012/140000

(56) Documents cités:
- US-A1- 2008 309 881
- US-A1- 2009 257 636
- US-A1- 2009 270 717
- BRANDON J. LUJAN ET AL.: "Revealing Henle's Fiber Layer Using Spectral Domain Optical Coherence Tomography", INVESTIGATIVE OPHTHALMOLOGY & VISUAL SCIENCE, vol. 52, no. 3, 1 mars 2011 (2011-03-01), pages 1486-1492, XP002658343, cité dans la demande

## Description

L'invention concerne un dispositif d'aide à la détection de caractéristiques anatomiques d'au moins une portion d'un tissu par exemple un tissu rétinien. L'invention concerne également un procédé d'aide à la détection de caractéristiques anatomiques d'au moins une portion d'un tissu.

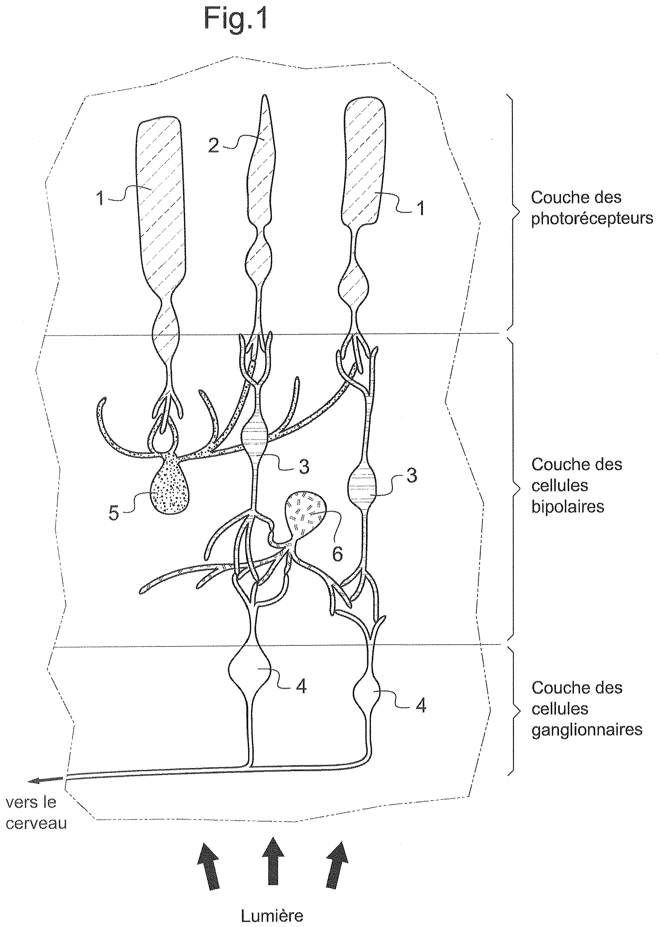

Une rétine est une fine membrane stratifiée de quelques dixièmes de millimètres. La figure 1 représente schématiquement une portion d'une rétine. De façon simplifiée, la rétine comporte trois couches principales. Dans la première couche, la rétine comporte des photorécepteurs ayant sensiblement la forme de bâtonnets 1 (pour la vision en noir et blanc et la vision nocturne) ou de cônes 2 (pour la vision en couleurs et la vision diurne), les photorécepteurs transformant des signaux lumineux en influx nerveux. Ces influx nerveux sont ensuite transmis à des cellules dites bipolaires 3 situées dans la deuxième couche. Les cellules bipolaires 3 communiquent les influx nerveux à des cellules dites ganglionnaires 4 situées dans la troisième couche. Des axones des cellules ganglionnaires 4 sont reliés au cerveau pour lui transmettre les influx nerveux, l'ensemble desdits axones formant un nerf appelé nerf optique. Les trois couches principales sont séparées par deux couches intermédiaires qui comportent des cellules participant à la régulation de la transmission des influx nerveux. Pour la première couche intermédiaire, des cellules dites horizontales 5 reçoivent les influx nerveux des photorécepteurs 1, 2 et les transmettent aux cellules bipolaires 3 adjacentes. Pour la deuxième couche intermédiaire, des cellules dites amacrines 6 reçoivent les influx nerveux des cellules bipolaires 3 et les transmettent aux cellules ganglionnaires 4 adjacentes.

Au sein de la rétine, des vaisseaux sanguins sont présents pour l'irrigation de ladite rétine. En outre, certaines structures de la rétine se présentent sous la forme de fibrilles sensiblement parallèles les unes aux autres comme les axones des photorécepteurs, les axones des cellules ganglionnaires, les segments externes des photorécepteurs...

### ARRIERE PLAN TECHNOLOGIQUE DE L'INVENTION

La Tomographie à Cohérence Optique ou TCO est une technique d'imagerie récente qui permet d'étudier des structures biologiques de façon très précise. La TCO autorise un examen non invasif d'une portion d'un tissu (tissu rétinien, muqueuse, épiderme...).

De façon simplifiée, la TCO est réalisée comme suit : un fin faisceau lumineux, le plus souvent infrarouge, issu d'une source est divisé en deux parties vers deux bras par une lame séparatrice. Dans un bras, la première partie du faisceau pénètre dans une portion du tissu à étudier pour être rétrodiffusée par ladite portion. Dans le deuxième bras dit de référence, la deuxième partie du faisceau est réfléchie par un miroir plan. Les deux parties du faisceau sont ensuite dirigées vers un interféromètre de Michelson qui permet d'extraire des franges d'interférences des deux parties du faisceau. Les franges d'interférences comportent des informations sur la portion de tissu étudiée. Par un balayage point par point du tissu étudié par la méthode que l'on vient de décrire, les informations recueillies sont combinées par des moyens de traitement qui retranscrivent alors une véritable coupe optique dudit tissu, ladite coupe ayant une résolution de l'ordre de quelques microns.

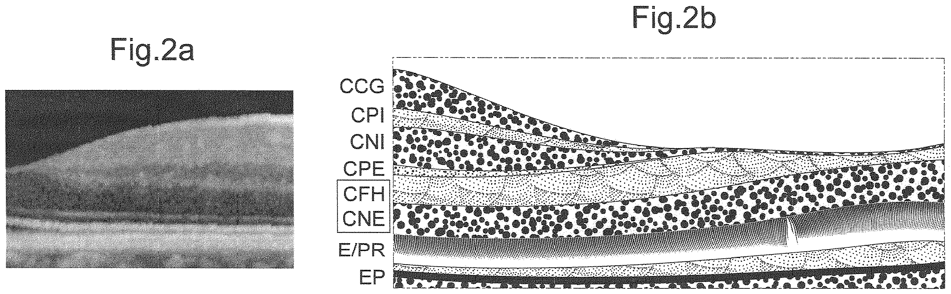

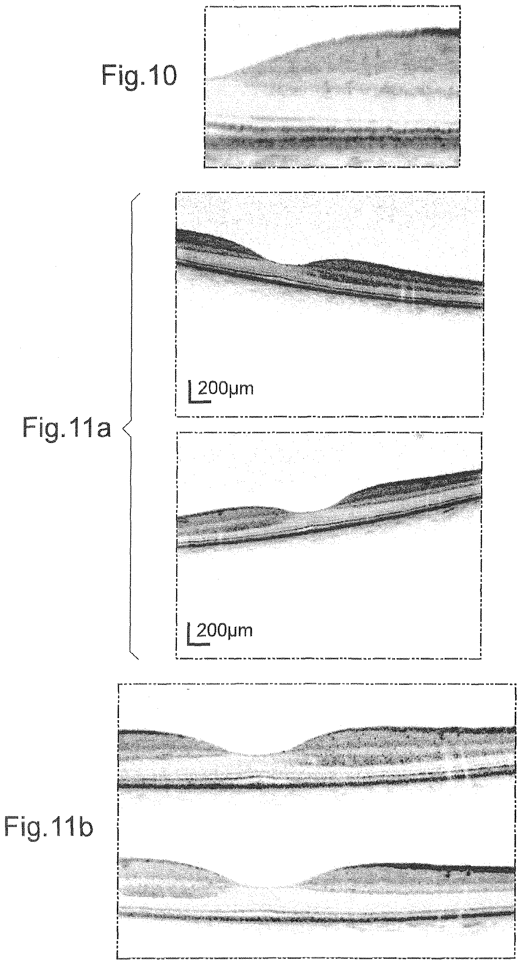

Dans le domaine de l'ophtalmologie, il est ainsi connu de réaliser un examen TCO pour étudier une rétine et notamment les différentes couches de la rétine. La figure 2a (ou la figure 10) est une image prise lors d'un examen TCO d'une rétine normale. La figure 2b illustre de façon schématique une observation au microscope d'une coupe histologique de la même rétine. Les différentes couches de la rétine se décomposent comme suit :
- Couche des Cellules Ganglionnaires (CCG) ou Ganglion Cell Layer (GCL) ;
- Couche Plexiforme Interne (CPI) ou Inner Plexiform Layer (IPL);
- Couche Nucléaire Interne (CNI) ou Inner Nuclear Layer (INL) ;
- Couche Plexiforme Externe (CPE) ou Outer Plexiform Layer (OPL) ;
- Couche des fibres de Henlé (CFH) ou Henle's Fiber Layer (HFL) ;
- Couche Nucléaire Externe (CNE) ou Outer Nuclear Layer (ONL) ;
- Couche des segments externes des photorécepteurs (E/PR) ou Outer Segment of the Photoreceptors layer (OS/PR) ;
- Epithélium Pigmentaire (EP) ou Retinal Pigment Epithelium (RPE).

Il a été constaté qu'un examen TCO de la rétine était bien plus précis qu'un examen classique comme une échographie de la rétine. Un examen TCO peut donc se révéler déterminant pour détecter un problème sur une rétine. Par exemple, un examen TCO permet de visualiser des lésions qui étaient jusqu'à là invisibles lors d'examens classiques. Toutefois, les lésions observées par TCO sont de nature très variées ce qui rend difficile une classification desdites lésions par leur description. Il s'avère donc difficile de détecter certaines lésions de façon automatique à partir des images prises par TCO.

De façon générale, l'interprétation des images prises par TCO repose uniquement sur l'intuition et l'expérience du médecin ce qui représente une lourde charge de travail pour ledit médecin.

L'article « Revealing Henle's Fiber Layer using Spectral Domain Optical Coherence Tomography » publié sur le site internet du journal Investigative Ophtalmology and Visual Science le 11 Novembre 2010 décrit une méthode pour identifier de façon précise la Couche des fibres de Henlé (CFH), couche qui comporte notamment les axones des photorécepteurs, lors d'un examen TCO. En effet, avec un examen TCO classique, il s'avère délicat de distinguer la Couche Nucléaire Externe (CNE) de la CFH. Il est ainsi expliqué dans ledit document que la réflexion de la CFH est dépendante de l'angle avec lequel un rayon pénètre la rétine lors d'un examen TCO, la CNE ne présentant pas ce trait distinctif. En choisissant un angle d'incidence adéquat, la réflexion de la CFH peut être très différente de celle de la CNE ce qui permet de les distinguer nettement. Il est même alors possible de mesurer l'épaisseur de la couche CFH.

L'article « Revealing Henle's Fiber Layer using Spectral Domain Optical Coherence Tomography » décrit un procédé permettant d'identifier clairement une couche entière et particulière de la rétine, à savoir la CFH. Ceci augmente la quantité d'information qu'il est possible d'obtenir d'images prises par TCO dans le cas d'une étude d'un tissu rétinien ce qui allège le travail d'interprétation des images par le médecin.

### OBJET DE L'INVENTION

Un but de l'invention est de simplifier encore davantage l'interprétation par un médecin d'images prises par TCO d'une portion d'un tissu, en aidant à la détection de caractéristiques anatomiques de ladite portion.

L'invention est applicable à tout type de tissus, notamment un tissu rétinien.

### BREVE DESCRIPTION DE L'INVENTION

En vue de la réalisation de ce but, on propose un dispositif d'aide à la détection de caractéristiques anatomiques d'au moins une portion d'un tissu, notamment d'un tissu rétinien, le dispositif comportant des moyens pour acquérir par tomographie à cohérence optique (TCO) des images de ladite portion prises selon des angles d'incidence distincts et des moyens de détection pour détecter sur les images, dans une même zone de la portion étudiée, au moins un motif ayant au moins une caractéristique qui varie d'une image à une autre, le motif étant susceptible d'être dû à la présence de caractéristiques anatomiques.

Les inventeurs se sont aperçus que deux images prises à des angles d'incidence distincts pouvaient présenter des motifs ayant chacun au moins une caractéristique qui variait d'une image à l'autre. La comparaison et l'analyse des deux images apportent ainsi une aide à la détection de caractéristiques anatomiques pouvant être reliées à de tels motifs.

Le dispositif selon l'invention augmente la quantité d'informations qu'il est possible d'obtenir d'images prises par TCO ce qui simplifie le travail d'interprétation des images par le médecin. Le médecin peut se concentrer sur la signification des motifs, leur lien avec des caractéristiques anatomiques voir les liens desdites caractéristiques anatomiques avec une éventuelle pathologie de la portion de tissu étudiée.

D'une image à l'autre c'est bien le même motif que l'on repère. Ledit motif est cependant seulement légèrement différent d'une image à une autre du fait de la variation d'au moins l'orientation du motif. On propose également un procédé d'aide à la détection de caractéristiques anatomiques d'au moins une portion d'un tissu, le procédé comportant les étapes de :
- acquérir par tomographie à cohérence optique au moins deux images de ladite portion prises selon des angles d'incidence distincts ;
- détecter sur les images dans une même zone de la portion étudiée, au moins un motif ayant au moins une caractéristique qui varie d'une image à une autre, le motif étant susceptible d'être dû à la présence de caractéristiques anatomiques.

### BREVE DESCRIPTION DES DESSINS

Les figures 1, 2a et 2b et 10 ont déjà été décrites et représentent la structure d'une rétine. La figure 10 est une vue en négatif (la balance des noirs et des blancs est inversée) de la figure 2a.

L'invention sera mieux comprise à la lumière de la description qui suit d'un mode de réalisation particulier non limitatif de l'invention en référence aux figures ci-jointes parmi lesquelles :
- la figure 3 est un schéma représentant les différentes étapes du procédé de l'invention ;
- les figures 4a, 4b illustrent chacune deux images d'une même portion d'un tissu rétinien, chaque vue ayant été acquise par TCO selon un angle d'incidence distinct, la figure 4a illustre les images brutes en sortie d'un examen TCO et la figure 4b les images après réorientation ;
- la figure 5a est une vue en coupe d'une rétine ;
- la figure 5b est un grossissement d'une partie de la coupe illustrée à la figure 3a ;
- la figure 5c est une représentation schématique des motifs présents dans la coupe illustrée aux figures 3a et 3b ;
- la figure 6 est une vue en coupe d'un tissu rétinien d'une personne malade;
- la figure 7 est un schéma représentant les différentes étapes d'une mise en oeuvre particulière du procédé de l'invention ;
- la figure 8 illustre trois images d'une même portion d'un tissu rétinien, chaque vue ayant été acquise par TCO selon un angle d'incidence distinct ;
- la figure 9 illustre trois images d'une même portion d'un tissu rétinien d'une personne malade, chaque vue ayant été acquise par TCO selon un angle d'incidence distinct.

La figure 11a est une vue en négatif de la figure 4a.

La figure 11b est une vue en négatif de la figure 4b.

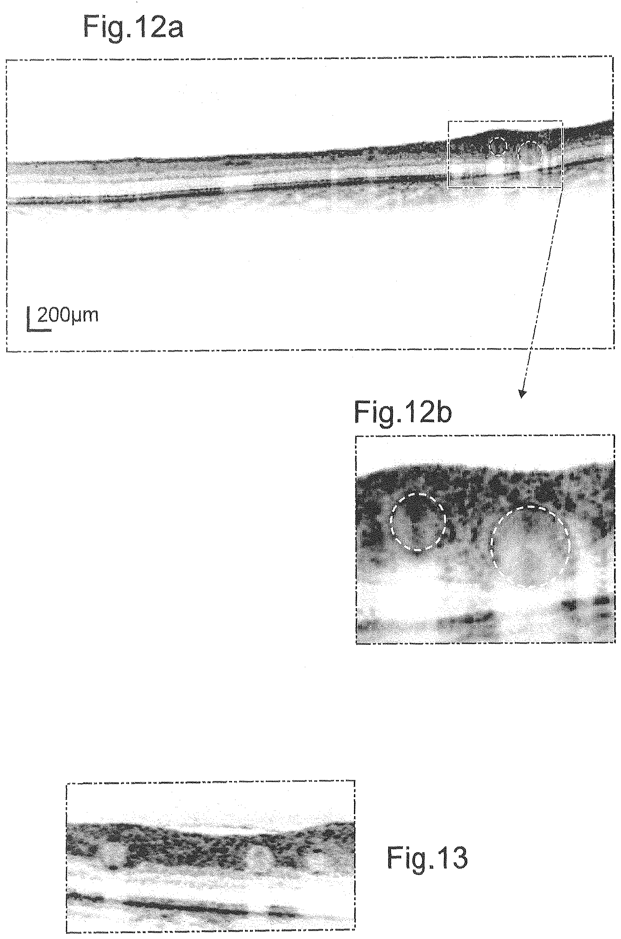

La figure 12a est une vue en négatif de la figure 5a.

La figure 12b est une vue en négatif de la figure 5b.

La figure 13 est une vue en négatif de la figure 6.

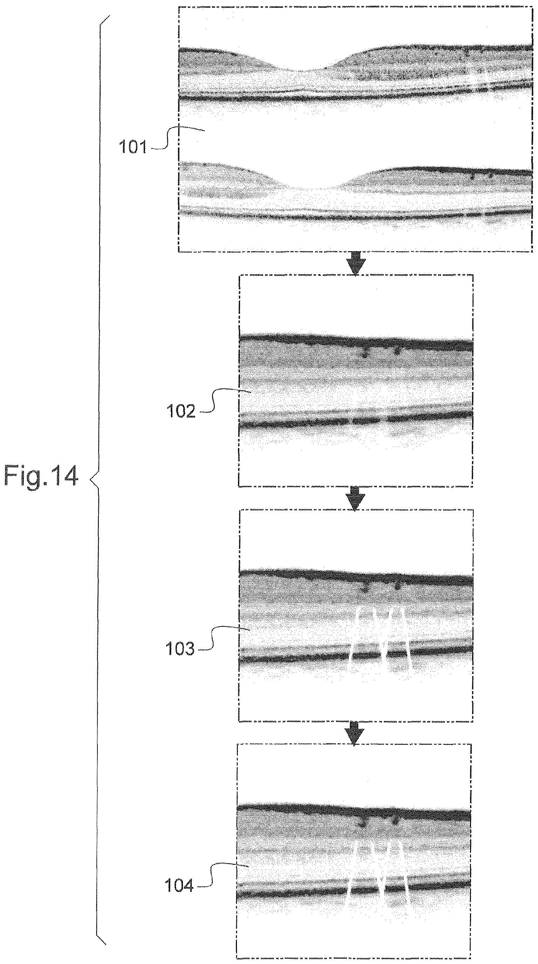

La figure 14 est une vue en négatif de la figure 7.

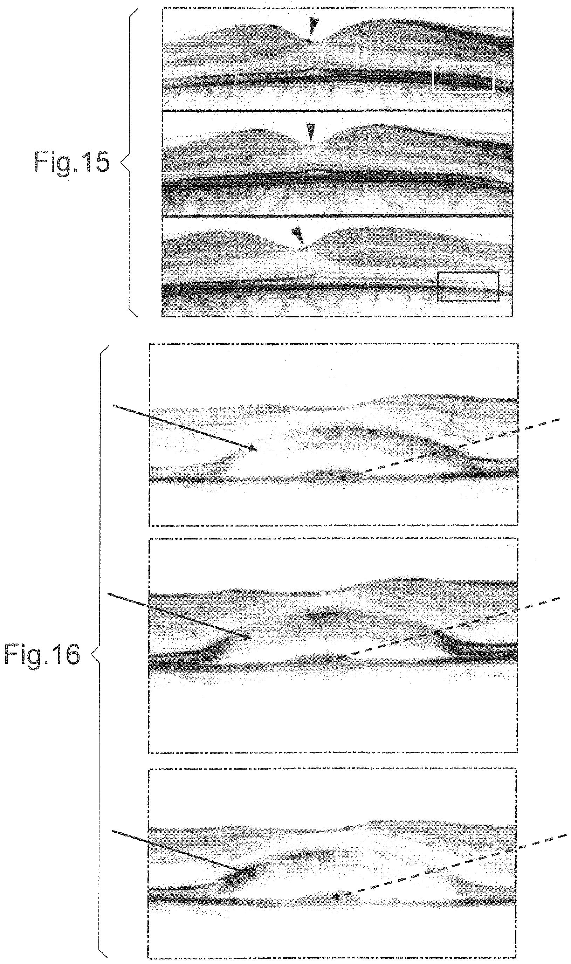

La figure 15 est une vue en négatif de la figure 8.

La figure 16 est une vue en négatif de la figure 9.

### DESCRIPTION DETAILLEE DE L'INVENTION

Le dispositif et le procédé d'aide à la détection de caractéristiques anatomiques de l'invention sont ici appliqués à une portion d'un tissu rétinien. Cette application n'est bien sûr pas limitative.

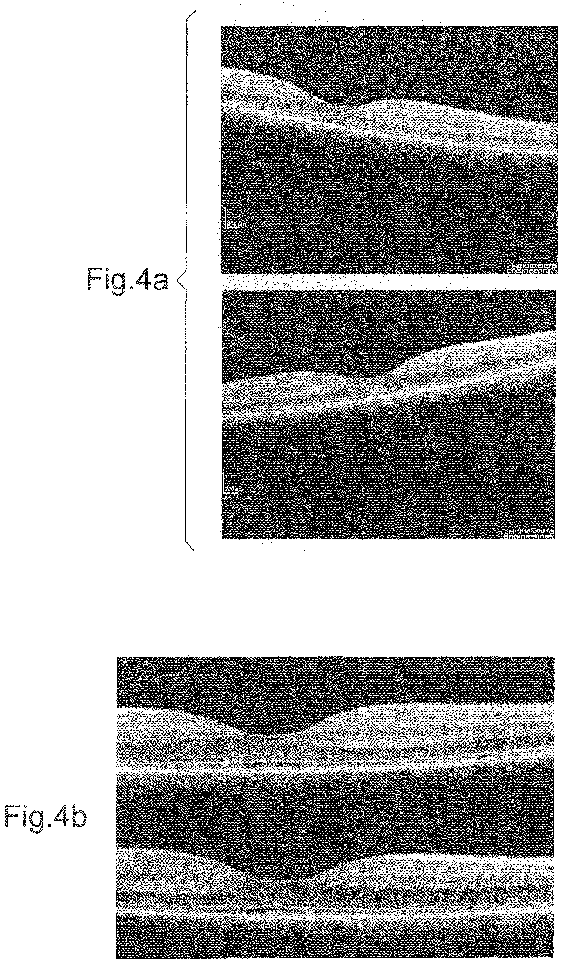

Selon l'invention, au moins deux images sont acquises par tomographie à cohérence optique (TCO) selon des angles d'incidence distincts. Pour la présente invention, les images illustrées à la figure 4a sont des images brutes en sortie d'examen par TCO qu'il convient de réorienter selon une même direction (comme illustrée à la figure 4b) avant de les comparer.

Le dispositif selon l'invention comporte des moyens pour acquérir par tomographie à cohérence optique (TCO) des images de ladite portion et des moyens de détection.

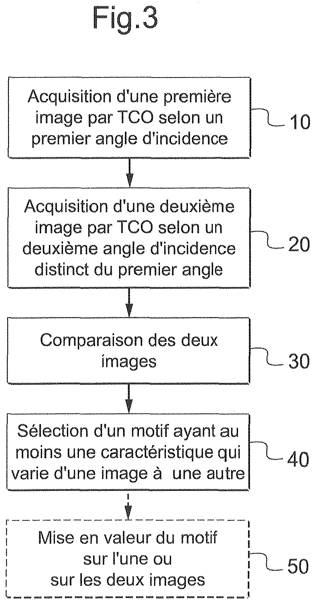

En référence à la figure 3, lors d'une première étape 10 du procédé selon l'invention, les moyens pour acquérir du dispositif de l'invention acquièrent une première image par Tomographie à Cohérence Optique ou TCO d'une portion d'un tissu rétinien. Ladite première image est prise selon un premier angle d'incidence. Dans un deuxième temps, lors d'une deuxième étape 20, les moyens pour acquérir du dispositif de l'invention acquièrent une deuxième image par TCO de la même portion de tissu rétinien, la deuxième image étant prise selon un deuxième angle d'incidence distinct du premier angle d'incidence. De façon connue en soi, dans le cas d'acquisition manuel des images par un médecin, il s'avère très aisé d'acquérir les images selon des angles d'incidences distincts. En effet, le médecin doit simplement incliner un palonnier en fonction de l'angle d'incidence avec lequel il souhaite acquérir l'image.

Les deux images sont ensuite comparées lors d'une troisième étape 30. Dans une quatrième étape 40, les moyens de détection détectent sur les deux images, dans une même zone de la portion étudiée, la présence d'un motif ayant au moins une caractéristique qui varie d'une image à une autre.

Selon un mode de réalisation particulier, le dispositif comporte des moyens de mise en valeur du motif. Dans un premier exemple de réalisation, les moyens de mise en valeur comportent des moyens pour entourer le motif détecté. Dans un deuxième exemple de réalisation, les moyens de mise en valeur comportent des moyens de coloration du motif. Ainsi, lors d'une cinquième étape 50 du procédé de l'invention, le motif est mis en valeur sur chaque image ou juste sur l'une des images. La cinquième étape 50 rend ainsi aisé, même pour une personne non issue du corps médical, la visualisation dudit motif.

Les inventeurs ont pû constater qu'un tel motif était susceptible d'être du à la présence de caractéristiques anatomiques. Les différentes étapes 10, 20, 30, 40, 50 du procédé décrites ci-dessus permettent ainsi d'aider à la détection de telles caractéristiques anatomiques.

De façon privilégiée, si aucun motif n'est détecté, une autre portion du tissu rétinien est analysée en reprenant les différentes étapes décrites ci-dessus à partir de la première étape d'acquisition 10.

Selon un premier mode de réalisation, on recherche plus particulièrement lors de la quatrième étape 40, un motif orienté différemment d'une image à une autre. La caractéristique du motif qui varie est donc ici l'orientation du motif. A cet effet, les moyens de détection du dispositif de l'invention comportent des moyens de recherche d'un motif orienté différemment d'une image à une autre.

En effet, les inventeurs ont pu identifier des motifs, susceptibles d'être dus à la présence de caractéristiques anatomiques, dont l'orientation est entièrement dépendante de l'angle d'incidence selon lequel sont acquises les images par TCO. Ceci facilite grandement la détection desdits motifs.

Quand bien même la Couche des fibres de Henlé ou CFH apparaît différemment d'une image à une autre du fait de ses propriétés optiques, elle demeure orientée de la même façon et n'entrave donc pas la détection des motifs.

Dans le cas d'un tissu rétinien, en moyenne, chaque angle d'incidence peut alors être choisi dans une plage d'environ -20 à 20 degrés par rapport à la direction d'un rayon lumineux traversant la rétine lors de l'examen TCO. La taille de l'oeil étudié et l'ouverture de la pupille associée peuvent modifier cette plage.

Selon un mode de réalisation privilégié de ce premier mode de réalisation, on recherche plus particulièrement, lors de la quatrième étape 40, un motif qui est orienté différent d'une image à une autre et qui est toujours orienté identiquement par rapport à la direction d'un rayon lumineux qui traverse la rétine. A cet effet, les moyens de détection du dispositif de l'invention comportent des moyens de recherche d'un motif orienté différemment d'une image à une autre et orienté identiquement par rapport à la direction d'un rayon lumineux qui traverse la rétine.

Les inventeurs ont pu mettre en évidence que l'interaction des vaisseaux sanguins ou des globules rouges avec le rayon lumineux qui traverse la rétine lors de l'examen TCO pouvait expliquer la présence de tels motifs. La détection desdits motifs permettrait ainsi de repérer des vaisseaux sanguins ou des globules rouges.

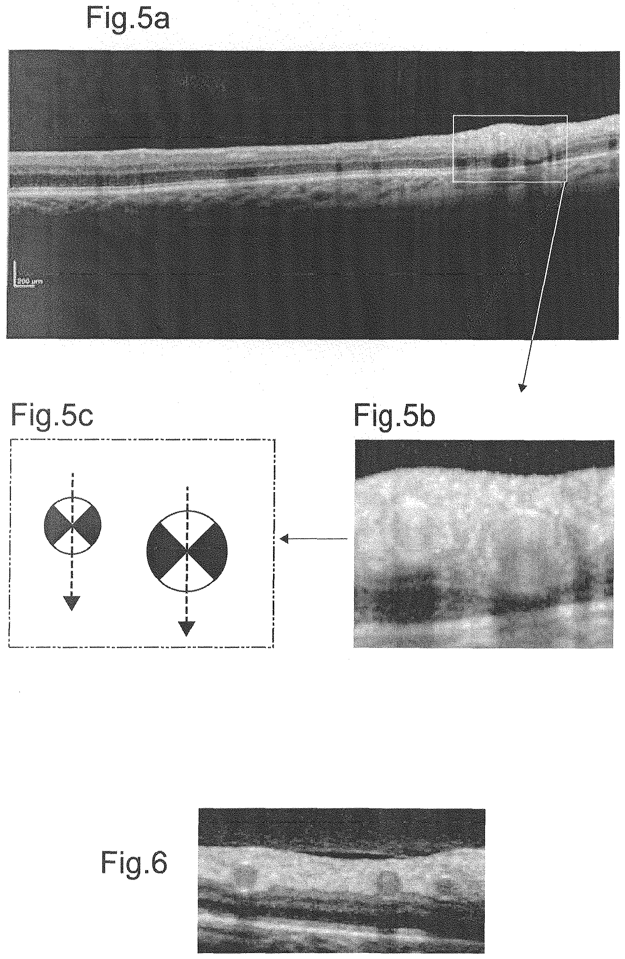

Selon une première variante de ce mode de réalisation privilégié, on recherche plus particulièrement un motif en forme de sablier. A cet effet, les moyens de recherche comportent des moyens d'identification d'un motif en forme de sablier. Comme illustré aux figures 5a, 5b et 5c, deux sabliers distincts sont identifiables dans l'une des couches du tissu rétinien. Comme schématisé à la figure 5c, l'orientation des sabliers est déterminée par l'angle d'incidence avec lequel le rayon lumineux (symbolisé par les flèches en pointillés) traverse la rétine lors de l'examen TCO. Un motif en sablier possède donc un aspect bien particulier : deux zones à forte réflectance (symbolisées en blanc à la figure 5c) sont situées symétriquement de part et d'autre du rayon lumineux. Ces deux zones sont séparées par deux autres zones plus sombres situées également symétriquement de part et d'autre du rayon lumineux.

Ainsi, lorsque l'angle d'incidence avec lequel est acquise la deuxième image est modifié, l'orientation des sabliers change en conséquence de sorte que les sabliers soient toujours orientés de la même manière par rapport au rayon lumineux.

D'une image à l'autre, seuls les sabliers sont orientés différemment ce qui facilite l'étape de détection desdits sabliers.

Les inventeurs ont pu mettre en évidence que les motifs en sablier étaient susceptibles d'être dus à la présence de vaisseaux sanguins, un tel sablier évoquant la section d'un vaisseau sanguin. Lorsqu'un rayon lumineux traverse un vaisseau, il est réfracté par des parois dudit vaisseau ce qui donne lieu à une représentation particulière en forme de sablier.

Selon une deuxième variante de ce mode de réalisation privilégié, on recherche plus particulièrement un motif qui est une ombre c'est-à-dire sensiblement un trait dont la réflectance est bien moins importante qu'une partie de tissu environnante. A cet effet, les moyens de recherche comportent des moyens d'identification d'un motif qui est une ombre. L'orientation de l'ombre est dépendante de l'angle d'incidence. Si l'angle d'incidence change, il y une variation correspondante de l'orientation de sorte que l'ombre soit toujours parallèle au rayon lumineux. D'une image à l'autre, seule l'ombre est orientée différemment ce qui facilite l'étape de détection de ladite ombre.

Les inventeurs ont pu mettre en évidence que les motifs qui sont des ombres étaient majoritairement des ombres de vaisseaux sanguins.

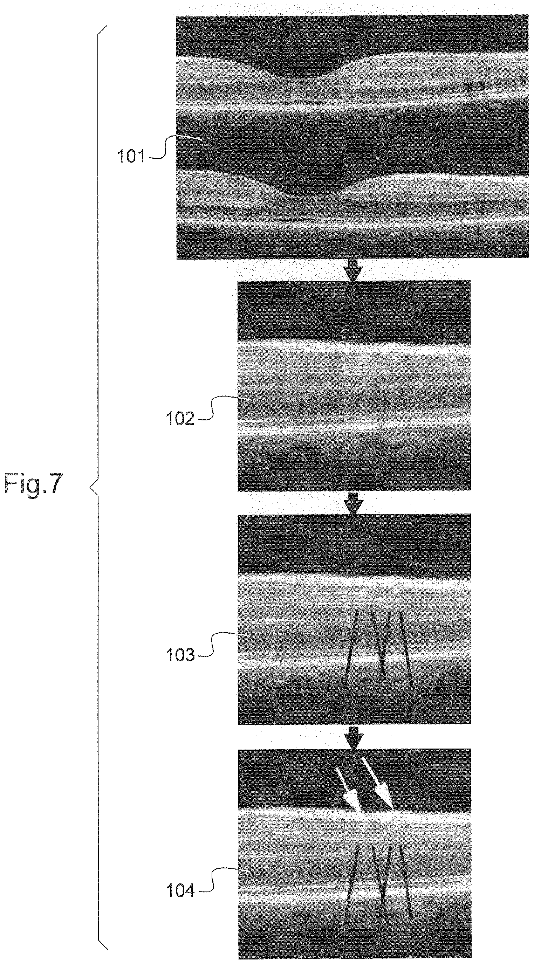

Selon un mode de réalisation particulier de cette deuxième variante, le dispositif selon l'invention comporte en outre des moyens de repérage d'un point focal. En référence à la figure 7, lors d'une première étape 101, deux images prises par TCO selon des angles d'incidences distincts et sur lesquelles deux ombres ont été détectées sont sélectionnées. Comme il est possible de le constater, chaque ombre est orientée différemment d'une image à une autre, une ombre étant toujours orientée de la même manière par rapport au rayon lumineux.

Lors d'une deuxième étape 102, des moyens de superposition du dispositif de l'invention superposent les deux images. Puis, dans une troisième étape 103, les moyens de repérage prolongent les ombres. Cette étape 103 est encore appelée étape de triangulation. Les ombres étant orientées différemment d'une image à une autre, leurs prolongations finissent par converger. Dans la quatrième étape 104, les moyens de repérage identifient le point de convergence qui est le point focal.

Les inventeurs ont pu constater que généralement ledit point focal était sensiblement proche voir confondu avec un vaisseau sanguin à l'origine de l'ombre. Ainsi, en identifiant le point focal, il est possible d'identifier un motif en forme de sablier proche ou confondu avec ledit point focal et qui soit représentatif dudit vaisseau sanguin.

Ainsi, non seulement le dispositif et le procédé de l'invention permettent de détecter un motif qui est une ombre sur les images mais ils permettent également de localiser le vaisseau sanguin qui est à l'origine de l'ombre.

Les inventeurs ont pu constater qu'il était plus aisé de détecter directement des motifs en forme de sablier lorsqu'ils sont susceptibles d'être dus à la présence de vaisseaux sanguins de taille importante. Lorsque les motifs en forme de sablier sont susceptibles d'être dus à la présence de vaisseaux sanguins de petites tailles, les inventeurs ont pu constater qu'il était plus aisé de détecter lesdits motifs en forme de sablier en passant par la détection des motifs en forme d'ombres associés.

Le dispositif et le procédé de l'invention simplifient ainsi grandement la tâche d'un médecin qui doit interpréter lesdites images.

Les inventeurs ont également constaté que les moyens de repérage pouvaient ne repérer aucun vaisseau sanguin à l'origine de l'ombre et que celle-ci devait dès lors être dûe à la présence d'une autre caractéristique anatomique. De façon plus générale, il est donc intéressant de rechercher une ombre pour pouvoir localiser une caractéristique anatomique à l'origine de l'ombre, ladite caractéristique anatomique pouvant être de petite taille. Ce cas de figure est cependant plus rare et ne se rencontre que dans le cas d'une rétine malade par exemple dans le cas d'une hémorragie de la rétine, d'une exsudation ...

Par ailleurs, les inventeurs ont découvert que la réfraction d'un rayon lumineux par le vaisseau était directement liée à la présence et à la concentration de globules rouges dans ledit vaisseau. En effet, les globules rouges exercent un fort pouvoir réfractif sur le rayon lumineux. Le motif en forme de sablier est donc représentatif de la concentration en globules rouges dans le vaisseau sanguin associé. La figure 6 illustre une rétine d'une personne atteinte d'anémie. Le motif en forme de sablier est ici nettement moins visible que pour une rétine normale (figures 5a, 5b et 5c). Ainsi, si les moyens de recherche ne peuvent identifier un motif en forme de sablier mais qu'un motif en forme d'ombre, il est possible de remonter par l'ombre à un point focal. Un médecin étudiant ledit point focal peut en déduire que le motif en forme de sablier n'est pas présent ou très atténué ce qui est significatif d'un faible taux de globules rouges et/ou d'une circulation sanguine alternée.

Selon un mode de réalisation particulier de cette deuxième variante, le dispositif selon l'invention comporte en outre des moyens de suppression de l'ombre pour créer une nouvelle image sur laquelle n'apparaît plus l'ombre ou une ombre marginale, que cette ombre soit due à un vaisseau sanguin ou à une autre caractéristique anatomique.

En effet, d'une image à une autre, l'ombre voit son orientation changer. De ce fait, d'une image à l'autre, un tronçon d'une région de la rétine contenant l'ombre se trouve dégagé et un autre tronçon est à son tour masqué par l'ombre. Ainsi, l'ombre ne recouvre jamais entièrement le même tronçon de ladite région. Les moyens de suppression récupèrent donc sur chaque image acquise un tronçon de ladite région qui ne soit pas recouvert par l'ombre et superposent lesdits tronçons de sorte à reconstruisent la région sans l'ombre ou avec une ombre marginale.

La région artificiellement reconstruite est alors réintégrée à une image de la portion étudiée. La nouvelle image ainsi modifiée est de bien meilleure qualité que les images comportant l'ombre, la région préalablement masquée par l'ombre étant à présent visible.

Selon un mode de réalisation privilégié, que l'on identifie un vaisseau sanguin soit par un sablier soit par une ombre, lors de la cinquième étape 50, les moyens de mise en valeur comportent des moyens pour colorer le vaisseau sanguin sur chaque image. Puis, si le tissu rétinien est étudié selon un autre axe de coupe, les moyens de mise en valeur colorent de nouveau le vaisseau sanguin sur les nouvelles images. Le dispositif de l'invention comporte en outre des moyens pour relier les différentes images de sorte qu'en colorant le vaisseau sanguin sur l'ensemble des coupes réalisées, on obtient ainsi une angiographie de ladite rétine.

Selon un deuxième mode de réalisation, on recherche plus particulièrement lors de la quatrième étape 40, un motif dont la réflectance est différente d'une image à une autre. La caractéristique du motif qui varie est donc ici la réflectance du motif. A cet effet, les moyens de détection du dispositif de l'invention comportent des moyens de recherche d'un motif dont la réflectance est différente d'une image à une autre.

Les inventeurs ont pu identifier des motifs, susceptibles d'être dus à la présence de caractéristiques anatomiques, dont la réflectance varie en fonction de l'angle d'incidence selon lequel sont acquises les images par TCO. Ceci facilite grandement la détection desdits motifs.

Les inventeurs ont pu constater que lesdits motifs présentent une anisotropie optique, la réflexion des motifs étant dépendante de l'angle d'incidence. Ceci explique que leur réflectance varie avec l'angle d'incidence.

Les inventeurs ont mis en évidence que lesdits motifs pouvaient se retrouver dans n'importe quelle zone de la rétine mais étaient situés le plus souvent dans la couche des segments externes des photorécepteurs (E/PR).

Quand bien même la Couche des fibres de Henlé ou CFH apparaît différemment d'une image à une autre du fait de ses propriétés optiques, elle représente une couche entière, continue et distincte de la rétine et est donc aisément distinguable des motifs situés dans des zones localisées de la portion étudiée.

Dans le cas d'un tissu rétinien, en moyenne, chaque angle d'incidence peut alors être choisi dans une plage d'environ -20 à 20 degrés par rapport à la direction d'un rayon lumineux traversant la rétine lors de l'examen TCO. La taille de l'oeil étudié et l'ouverture de la pupille associée peuvent modifier cette plage.

Selon un mode de réalisation privilégié, les moyens de coloration prennent en considération la réflectance du motif pour colorer plus ou moins fortement ledit motif d'une image à une autre et ainsi illustrer l'influence de l'angle d'incidence sur la réflectance dudit motif.

De façon privilégiée, le dispositif selon l'invention comporte en outre des moyens de sélection. De la même façon, le procédé selon l'invention comporte une étape supplémentaire qui consiste à sélectionner l'image sur laquelle le motif est le plus clair par l'intermédiaire des moyens de sélection.

De préférence, le dispositif selon l'invention comporte des moyens de calcul d'un angle d'incidence pour lequel ledit motif a une réflectance maximale. A cet effet, les moyens de calcul déterminent la réflectance du motif sur chaque image et l'associent à l'angle d'incidence selon lequel a été acquise l'image. Puis, les moyens de calcul estiment un angle d'incidence pour lequel ledit motif à une réflectance maximale.

Bien entendu, plus le nombre d'images acquis est important, plus l'estimation de l'angle de la réflectance maximale par les moyens de calcul sera précise.

Selon un mode de réalisation particulier, on recherche plus particulièrement un motif en forme de ligne. A cet effet, les moyens de recherche comportent des moyens d'identification d'un motif qui est une ligne. La réflectance de ladite ligne est dépendante de l'angle d'incidence. Si l'angle d'incidence change, il y une variation correspondante de la réflectance de la ligne. D'une image à l'autre, seule la ligne est d'une réflectance différente ce qui facilite l'étape de détection des lignes.

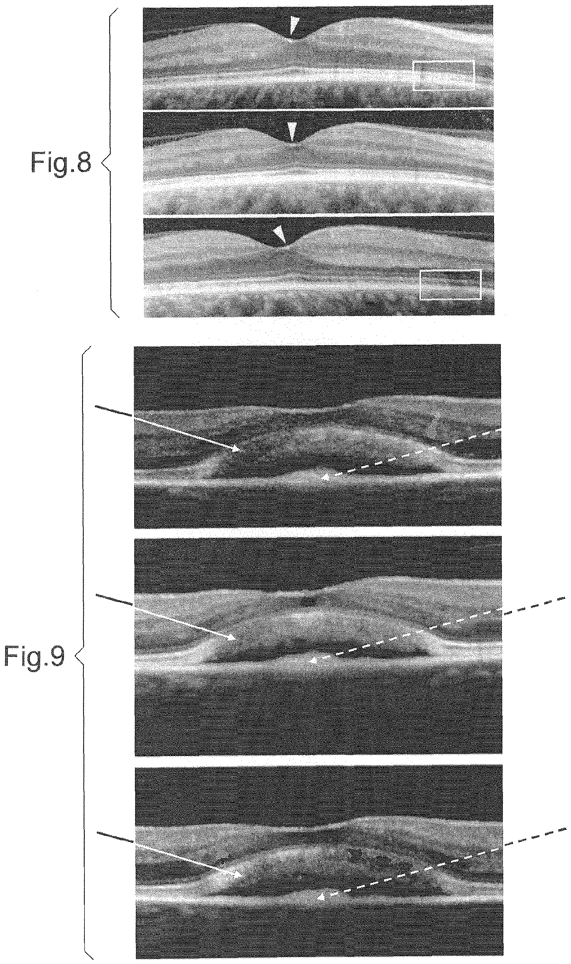

Comme il a déjà été indiqué certaines structures de la rétine se présentent sous la forme de fibrilles sensiblement parallèles les unes aux autres comme les axones des photorécepteurs, les axones des cellules ganglionnaires, les segments externes des photorécepteurs ... Les motifs en forme de ligne sont susceptibles d'être dus à la présence de ses structures. Les inventeurs ont remarqué qu'un motif en forme de ligne pouvait même être directement l'une de ses fibrilles. En référence à la figure 8, il est ainsi clairement visible que la zone encadrée par un rectangle blanc a une réflectance différente selon l'angle d'incidence avec lequel un rayon lumineux traverse la rétine (symbolisé par la flèche blanche). Cette zone comporte des segments externes des photorécepteurs.

En référence à la figure 9, un dépôt (flèche en pointillée) s'est accumulé sur la rétine d'une personne. Alors que d'une image à l'autre, le dépôt garde une réflectance identique, un motif (flèche pleine) dû à des segments externes de photorécepteurs a une réflectance différente. Il est ainsi possible de distinguer très facilement le dépôt des segments externes.

De façon privilégiée, le dispositif selon l'invention comporte des moyens de repérage. Après sélection de l'image sur lequel la ligne est la plus claire, on détermine dans une étape ultérieure, une direction selon laquelle s'étend ladite ligne par l'intermédiaire des moyens de repérage sur l'image sur laquelle la ligne est la plus claire.

Les inventeurs ont pu mettre en évidence que lorsque la ligne est la plus claire, c'est-à-dire lorsque sa réflectance est la plus importante, la direction de ladite ligne était susceptible d'indiquer une direction d'orientation préférentielle d'une structure se présentant sous la forme de fibrilles et se situant dans une région adjacente à ladite ligne. La détermination de la direction d'orientation préférentielle permet ainsi de déterminer la direction selon laquelle sont globalement parallèles lesdites fibrilles.

Le dispositif et le procédé selon l'invention permettent d'extraire une importante quantité d'informations d'images prises par TCO. Pour un tissu rétinien, il est ainsi notamment possible de détecter des motifs qui sont susceptibles d'être dus à la présence de vaisseaux sanguins et de fibrilles que ce soit pour un tissu normal ou un tissu malade.

Bien entendu l'invention n'est pas limitée au mode de réalisation et à la mise en oeuvre décrits et on peut y apporter des variantes de réalisation sans sortir du cadre de l'invention tel que défini par les revendications.

En particulier, bien qu'ici le dispositif et le procédé ait été décrits en application à un tissu rétinien, on peut avoir recours au dispositif et/ou au procédé de l'invention pour la détection de caractéristiques anatomiques d'au moins une portion d'un autre tissu susceptible d'être analysé par TCO comme une cornée, une muqueuse ou une portion d'épiderme par exemple. Le dispositif et/ou le procédé sera appliqué de préférence à des tissus de faible épaisseur, c'est-à-dire de quelques millimètres au maximum. La détection de motifs en forme de sablier ou également d'ombres qui sont susceptibles d'être dus à la présence de vaisseaux sanguins peut bien sûr être réalisée pour d'autres tissus qu'un tissu rétinien.

Bien qu'ici seulement deux images soient acquises par TCO pour aider à la détection de caractéristiques anatomiques d'au moins une portion d'un tissu, plus d'images pourront être utilisées. De plus, plusieurs motifs pourront être détectés en même temps même si les motifs sont tous différents.

## Revendications

1. Dispositif d'aide à la détection de caractéristiques anatomiques d'au moins une portion d'un tissu, notamment d'un tissu rétinien, le dispositif comportant des moyens pour acquérir par tomographie à cohérence optique (TCO) des images de ladite portion prises selon des angles d'incidence distincts et des moyens de détection pour détecter sur les images, dans une même zone de la portion étudiée, au moins un motif ayant au moins une caractéristique qui varie d'une image à une autre, le motif étant susceptible d'être dû à la présence de caractéristiques anatomiques, **caractérisé en ce que** les moyens de détection comportent des moyens de recherche d'un motif orienté différemment d'une image à une autre.

2. Dispositif d'aide à la détection selon la revendication 1, dans lequel les moyens de détection comportent des moyens de recherche d'un motif orienté différemment d'une image à une autre et qui est toujours orienté identiquement par rapport à une direction d'un rayon lumineux qui traverse le tissu pour l'acquisition des images.

3. Dispositif d'aide à la détection selon la revendication 2, dans lequel les moyens de recherche comportent des moyens d'identification d'un motif en forme de sablier.

4. Dispositif d'aide à la détection selon la revendication 2, dans lequel les moyens de recherche comportent des moyens d'identification d'un motif qui est une ombre.

5. Dispositif d'aide à la détection selon la revendication 4, comportant en outre des moyens de suppression de l'ombre dans une région qui comporte l'ombre par récupération d'un tronçon de ladite région qui ne soit pas recouvert par l'ombre sur chaque image acquise et par superposition desdits tronçons.

6. Dispositif d'aide à la détection selon la revendication 4, comportant des moyens de repérage d'un point focal par prolongation des ombres et identification de leur convergence sur des images superposées.

7. Dispositif d'aide à la détection selon la revendication 1, dans lequel les moyens de détection comportent en outre des moyens de recherche d'un autre motif dont la réflectance est différente d'une image à une autre.

8. Dispositif d'aide à la détection selon la revendication 7, comportant des moyens de sélection de l'image sur laquelle l'autre motif est le plus clair.

9. Dispositif d'aide à la détection selon la revendication 7, dans lequel les moyens de recherche comportent des moyens d'identification d'un autre motif en forme de ligne.

10. Dispositif d'aide à la détection selon les revendications 8 et 9, comportant des moyens de repérage d'une direction selon laquelle s'étend ladite ligne sur l'image sur laquelle la ligne est la plus claire.

11. Dispositif d'aide à la détection selon la revendication 1, comportant en outre des moyens de mise en valeur du motif sur une ou plusieurs images.

12. Dispositif d'aide à la détection selon la revendication 11, dans lequel les moyens de mise en valeur comportent des moyens pour entourer le motif.

13. Dispositif d'aide à la détection selon la revendication 12, dans lequel les moyens de mise en valeur comportent des moyens de coloration du motif.

14. Procédé d'aide à la détection de caractéristiques anatomiques d'au moins une portion d'un tissu, notamment d'un tissu rétinien, le procédé comportant les étapes de :
- acquérir par tomographie à cohérence optique (TCO) au moins deux images de ladite portion prises selon des angles d'incidence distincts ;
- détecter sur les images dans une même zone de la portion étudiée, au moins un motif ayant au moins une caractéristique qui varie d'une image à une autre, le motif étant susceptible d'être dû à la présence de caractéristiques anatomiques, au moins une caractéristique étant l'orientation du motif.

## Patentansprüche

1. Vorrichtung zur Unterstützung bei der Detektion von anatomischen Eigenschaften mindestens eines Abschnittes eines Gewebes, insbesondere eines Netzhautgewebes, wobei die Vorrichtung Mittel umfasst, um mittels optischer Kohärenztomografie (OCT) Bilder des genannten Abschnittes zu erfassen, die gemäß unterschiedlichen Einfallswinkeln aufgenommen wurden, sowie Detektionsmittel, um auf den Bildern innerhalb einer selben Zone des untersuchten Abschnittes mindestens ein Motiv zu detektieren, das mindestens eine Eigenschaft hat, die von einem Bild zum anderen variiert, wobei das Motiv dazu geeignet ist, auf das Vorhandensein von anatomischen Eigenschaften zurückgeführt zu werden, **dadurch gekennzeichnet, dass** die Detektionsmittel Suchmittel zum Suchen eines Motivs umfassen, das von einem Bild zum anderen unterschiedlich ausgerichtet ist.

2. Vorrichtung zur Unterstützung bei der Detektion nach Anspruch 1, bei der die Detektionsmittel Suchmittel zum Suchen eines Motivs umfassen, das von einem Bild zum anderen unterschiedlich ausgerichtet ist und das in Bezug auf eine Richtung eines Lichtstrahls, der zur Erfassung der Bilder durch das Gewebe hindurchgeht, immer identisch ausgerichtet ist.

3. Vorrichtung zur Unterstützung bei der Detektion nach Anspruch 2, bei der die Suchmittel Identifizierungsmittel zur Identifizierung eines Motivs in Form einer Sanduhr umfassen.

4. Vorrichtung zur Unterstützung bei der Detektion nach Anspruch 2, bei der die Suchmittel Identifizierungsmittel zur Identifizierung eines Motivs umfassen, das ein Schatten ist.

5. Vorrichtung zur Unterstützung bei der Detektion nach Anspruch 4, ferner umfassend Unterdrückungsmittel zur Unterdrückung des Schattens in einem den Schatten umfassenden Bereich mittels Wiederverwendung eines Abschnittes des genannten Bereichs, der nicht von dem Schatten überdeckt ist, in jedem erfassten Bild und durch Überlagerung der genannten Abschnitte.

6. Vorrichtung zur Unterstützung bei der Detektion nach Anspruch 4, umfassend Ortungsmittel zum Orten eines Brennpunktes mittels Verlängerung der Schatten und Identifizierung ihrer Konvergenz auf den überlagerten Bildern.

7. Vorrichtung zur Unterstützung bei der Detektion nach Anspruch 1, bei der die Detektionsmittel ferner Suchmittel zur Suche eines weiteren Motivs umfassen, dessen Reflexionsgrad von einem Bild zum anderen unterschiedlich ist.

8. Vorrichtung zur Unterstützung bei der Detektion nach Anspruch 7, umfassend Auswahlmittel zum Auswählen des Bildes, auf dem das andere Motiv das hellste ist.

9. Vorrichtung zur Unterstützung bei der Detektion nach Anspruch 7, bei der die Suchmittel Identifizierungsmittel zum Identifizieren eines anderen Motivs in Linienform umfassen.

10. Vorrichtung zur Unterstützung bei der Detektion nach den Ansprüchen 8 und 9, umfassend Ortungsmittel zum Orten einer Richtung, gemäß der sich die genannte Linie auf dem Bild erstreckt, auf dem die Linie die hellste ist.

11. Vorrichtung zur Unterstützung bei der Detektion nach Anspruch 1, ferner umfassend Hervorhebungsmittel zum Hervorheben des Motivs auf einem oder mehreren Bildern.

12. Vorrichtung zur Unterstützung bei der Detektion nach Anspruch 11, bei der die Hervorhebungsmittel Mittel zum Einkreisen des Motivs umfassen.

13. Vorrichtung zur Unterstützung bei der Detektion nach Anspruch 12, bei der die Hervorhebungsmittel Einfärbungsmittel zum Einfärben des Motivs umfassen.

14. Verfahren zur Unterstützung bei der Detektion von anatomischen Eigenschaften mindestens eines Abschnittes eines Gewebes, insbesondere eines Netzhautgewebes, wobei das Verfahren die Schritte umfasst:
- Erfassen mittels optischer Kohärenztomographie (OCT) von mindestens zwei Bildern des genannten Abschnittes, die gemäß unterschiedlichen Einfallswinkeln aufgenommen wurden;
- Detektieren auf den Bildern in einer selben Zone des untersuchten Abschnittes mindestens eines Motivs, das mindestens eine Eigenschaft hat, die von einem Bild zum anderen variiert, wobei das Motiv dazu geeignet ist, auf das Vorhandensein von anatomischen Eigenschaften zurückgeführt zu werden, wobei mindestens eine Eigenschaft die Ausrichtung des Motivs ist.

## Claims

1. A device for aiding with detection of anatomical features in at least one portion of a tissue, especially a retinal tissue, the device comprising means for acquiring, by optical coherence tomography (OCT), images of said portion taken at separate angles of incidence, and detecting means for detecting in the images, in a given region of the studied portion, at least one pattern having at least one characteristic that varies from one image to another, the pattern being liable to be due to the presence of anatomical features, being **characterized in that** the detecting means comprise means for searching for a pattern oriented differently from one image to another.

2. The device for aiding with detection as claimed in claim 1, the detecting means comprise means for searching for a pattern oriented differently from one image to another, which pattern is always oriented identically relative to a direction of a light beam that passes through the tissue for the purpose of image acquisition.

3. The device for aiding with detection as claimed in claim 2, in which the searching means comprise means for identifying an hourglass-shaped pattern.

4. The device for aiding with detection as claimed in claim 2, in which the searching means comprise means for identifying a pattern that is a shadow.

5. The device for aiding with detection as claimed in claim 4, furthermore comprising means for removing the shadow from a region that comprises the shadow by retrieving a section of said region, which section is not covered by the shadow, from each acquired image and superposing said sections.

6. The device for aiding with detection as claimed in claim 4, comprising means for locating a focal point by extending the shadows and identifying the point where they converge on the superposed images.

7. The device for aiding with detection as claimed in claim 1, in which the detecting means comprise also means for searching for another pattern the reflectance of which is different from one image to another.

8. The device for aiding with detection as claimed in claim 7, comprising means for selecting the image in which the other pattern is brightest.

9. The device for aiding with detection as claimed in claim 7, in which the searching means comprise means for identifying another pattern that is line-shaped.

10. The device for aiding with detection as claimed in claims 8 and 9, comprising means for locating a direction in which said line extends in the image in which the line is the brightest.

11. The device for aiding with detection as claimed in claim 1, furthermore comprising means for highlighting the pattern in one or more images.

12. The device for aiding with detection as claimed in claim 11, in which the highlighting means comprise means for encircling the pattern.

13. The device for aiding with detection as claimed in claim 12, in which the highlighting means comprise means for coloring the pattern.

14. A method for aiding with detection of anatomical features in at least one portion of a tissue, especially a retinal tissue, the method comprising steps of:
- acquiring, by optical coherence tomography (OCT), at least two images of said portion taken at separate angles of incidence; and
- detecting in the images, in a given region of the studied portion, at least one pattern having at least one characteristic that varies from one image to another, the pattern being liable to be due to the presence of anatomical features, at least one characteristic being the orientation of the pattern.
